# EUROPEAN PATENT SPECIFICATION

(11) **EP 0 206 575 B1**
(45) Date of publication and mention of the grant of the patent: **24.02.1993**
(21) Application number: 86304213.1
(22) Date of filing: 03.06.1986
(51) Int. Cl.: C01G 23/00, C01G 25/00, C01B 35/12

(54) **Process for making fine powders**
Verfahren zur Herstellung feiner Pulver
Procédé de préparation de poudres fines

(30) Priority: 17.06.1985 US 745045
(43) Date of publication of application: 30.12.1986
(73) Proprietor: MRA LABORATORIES, INC., North Adams, Massachusetts 01247 (US)
(72) Inventor: Cipollini, Ned Edward, New Jersey NJ 07041 (US)
(74) Representative: Spencer, Graham Easdale

(56) References cited:
- US-A- 3 250 832
- US-A- 3 647 364
- US-A- 4 293 514
- US-A- 4 487 755

## Description

The present invention is concerned with a process for making fine powders.

DE-A-2032233 describes a process for making finely divided ceramic materials which comprises emulsifying an aqueous solution of a metal salt in a water-immiscible liquid. The water of the emulsion is then evaporated without boiling away a significant part of the water-immiscible fluid and the emulsion is converted into a metal salt sol. The sol is then induced to coagulate, i.e. flocculate, for example, by heating and/or by addition of propanol, ethanol, or the like. The heating also serves to simultaneously decompose the metal salt to produce a refractory powder.

Another prior art process is described in US-A-3647364 and is directed to making Group I and Group II metal titanate powders. This process involves forming alcoholates of each of the precursor metals, mixing the alcoholates and refluxing the mixture, adding water to the resulting mixture to form a precipitate, separating the precipitate from the solution, and drying the recovered titanate powder.

The two processes described above by which particles of metal compounds are precipitated from a solution can produce fine powders, but inevitably some of the fine particles so produced agglomerate to form large particles.

We have now developed a process which is suitable for making finely divided powders having a wide range of possible compositions. Furthermore, the size of the particles produced by the process can be controlled and agglomeration of the resulting particles is prevented by maintaining particle separation throughout the process.

According to the present invention, there is provided a process for making a material in powder form, which comprises
(a) mixing an aqueous solution of a compound or compounds capable of conversion to the desired final product, the compound/compounds having a higher boiling point and decomposition temperature than the boiling point of water under the conditions used in step (b) below, and a water-immiscible organic liquid to form a water/organic liquid emulsion in which the aqueous solution is the disperse phase.
(b) heating the emulsion from step (a) to evaporate and remove the water and a portion of the organic liquid to give a suspension of solid particles constituting the precursor compound(s) in the remaining organic liquid,
(c) heating the suspension from step (b) in a non-oxidising atmosphere to char the organic liquid and form a carbon char matrix having the solid particles dispersed therein, and
(d) heating the product of step (c) in an oxidising atmosphere to oxidise and remove the carbon of the matrix and to leave the solid particles in the form of a powder.

For a better understanding of the invention, reference will be made in the following description to the accompanying drawings, in which:
Figure 1 shows a particle-size histogram of particles obtained at the dried emulsion stage of a process according to the invention for making barium titanate powder;
Figure 2 shows a particle-size histogram of particles at the char stage of the process;
Figure 3 shows a particle-size histogram of barium titanate particles at the oxidized/calcined stage;
Figure 4 shows a photograph of an S.E.M. image of the powder particles referred to in Figure 2;
Figure 5 shows a photograph of an S.E.M. image of the powder particles referred to in Figure 3; and
Figure 6 shows a graph relating to the calcination of oxidized powder, in which powder weight is plotted as a function of the heating temperature.

As indicated, the process of the present invention for making fine powders comprises preparing an aqueous solution of a compound or compounds capable of conversion to the desired powder product and then forming an emulsion of the aqueous solution in a water-immiscible organic liquid. The emulsion is heated to remove the water from the droplets, thereby converting the emulsion to a suspension of solid particles composed of the precursor compounds. Heating is continued to evaporate and remove a substantial proportion of the organic liquid. A subsequent heating is then performed in an essentially inert atmosphere to decompose the remainder of the organic liquid to give a carbon char matrix in which the separation of the particles is preserved. Finally, the carbon in the char matrix is removed by heating the matrix in a controlled oxygen atmosphere to oxidise the carbon to gaseous CO and CO₂, thereby leaving only the particles in the form of a powder.

The process of the invention can be used to produce fine powders of great compositional variety. It is particularly well suited for making powders of metal oxides, glasses, and ceramics for which water-soluble precursors are widely available. It is anticipated that pure metal powders can be made by such a process, particularly silver, palladium, platinum, and gold which are useful for making very thin precious metal connectors in electronic components.

Success in making fine powder particles by the method according to the invention depends upon taking particular measures during the removal of the water and the oxidation step following charring of the organic liquids to ensure that neither the emulsion droplets or the solid particles obtained in the later steps are able to combine. The finer the particles become, the more difficult this is to achieve, because smaller particles have a higher surface energy and therefore a greater tendency to agglomerate.

The tendency for the emulsion droplets to flocculate and/or sediment is reduced by heating the emulsion slowly when removing the water. This step of the process is preferably initially carried out at a temperature above the boiling point of water and at or below the boiling point of the organic liquid and then at a temperature at or above the boiling point of the organic liquid.

The tendency for the particles to agglomerate during the exothermic oxidation step is reduced by oxidising the matrix at a temperature below the characteristic minimum reaction or calcining temperature of the precursor compounds of which the particles are comprised. Such temperature control can be achieved by controlling the amount of oxygen in the oxidising atmosphere, by heating only thin sections of the char, and/or by reducing the rate of heating.

The invention relies centrally upon the charring step during which separation of the particles is preserved by the residue of carbon generated during this step. The fine powder product is thus made up of particles each of which arises directly from a droplet in the original emulsion.

The aqueous droplets in the emulsion contain the precursor compounds in the ratio required to produce the desired composition in the final powder product. Thus, it is possible using the process of the invention to achieve precise control of the final powder composition, an advantage which is particularly important when making ceramic powders for electrical applications. Furthermore, the present invention requires little, if any, comminution steps, which tend to introduce impurities in powder compositions made by conventional methods involving grinding, mixing, calcining, and further grinding steps. In addition, the precursor compounds are dissolved, and therefore very intimately mixed, in the aqueous solution. Thus not only is control of chemical composition, i.e. stoichiometry, more exactly effected, but the method of the present invention provides a purer product of unsurpassed compositional homogeneity.

The powder obtained by the process of the invention may be very fine and have a narrow particle size distribution. Average particle size and size distribution depend respectively upon the nominal size and size distribution of the droplets in the emulsion. The powder particles made by the present process are inherently spherical. Spherical particles having a narrow size distribution are considered to be ideal for obtaining the high packing densities which are required for making dense ceramic and metal films in the electronics industry.

The present invention thus provides a unique process whereby water-soluble precursor compounds are emulsified in an organic liquid which is subsequently heated to remove water from the emulsion droplets and then further heated in an essentially inert atmosphere to form a char consisting of the decomposed organic liquid. The carbon in the char maintains the separation between the dried droplet-derived particles. The char is then heated in air or in a controlled oxygen atmosphere to oxidize and remove the carbon and, optionally, to calcine the material to controllably produce a fine powder.

In order that the invention may be more fully understood, the following examples are given by way of illustration.

### Example 1

### (i) Preparation of a titanium citrate solution

1500ml. of doubly deionized water was placed in a 4 litre beaker and 1320g of anhydrous citric acid was added with stirring. The pH of the solution was brought to 4.0 by the addition of NH₄OH (about 750 ml). 1500 ml of tetra-isopropyl titanate (TPT) was then added at a rate of about 100 ml per minute. As the TPT was added, a white precipitate formed which gradually disappeared with stirring. The solution was then slowly heated to a temperature slightly above the boiling point of isopropanol (82.4°C) and was maintained at this temperature with stirring for about 6 hours in order to remove the isopropanol. The resulting solution was then cooled to room temperature and the pH adjusted to about 5.0. The solution was then diluted to 3000 ml by the addition of more doubly deionized water and filtered through a medium grade filter paper to remove any undissolved particles. The resulting solution of titanium citrate had a molar ratio of citrate to titanium of 1.30.

The solution can be assayed for TiO₂ content, for example, by ignition at 900°C for 2 hours. The TiO₂ content will be close to 11.00% by weight and this level of TiO₂ is assumed in the following examples illustrating the use of this solution.

### (ii) Preparation of a barium titanium citrate solution

3117.4 g of the titanium citrate solution obtained above was placed in a beaker and 1500 ml of doubly deionized water was added. 756.46g of anhydrous citric acid was then added with stirring and dissolved in the solution. The solution was heated to 40°C and 847.08 g of barium carbonate was slowly added. The barium carbonate reacted with the citrate solution to form a clear liquid, which was then cooled to room temperature. The pH was adjusted to 5.4 using NH₄OH and the solution diluted until a density of 1.22 gm/cm³ was obtained. The resulting solution of barium titanium citrate had a molar ratio of citrate to barium titanate of 2.22. Water was added to the solution to give a concentration of 0.1 mole of barium-titanium citrate per 175 ml of solution. The total volume obtained was about 7510 ml.

### (iii) Preparation of the oil phase

14.897 litres of ISOPAR M (Trade Mark) isoparaffin solvent was poured into a large container. This solvent has a boiling range of from 207°C to 260°C.

To the ISOPAR M was added 2.629 litres of DRAKEOL # 35 (Trade Mark), a high boiling mineral oil comprising a mixture of paraffin and naphthene and having a lower boiling point of 346°C.

350.52 g of a surfactant, or emulsifying agent, was added to the contents of the container, which were then thoroughly mixed. This amount of surfactant corresponded to 20 g per litre of organic liquid (oil). The particular surfactant used was OLOA 1200 (Trade Mark), a derivative of succinimide and polybutane.

### (iv) Emulsification

7510 ml of the barium titanium citrate solution obtained above was added to 17800 ml of the oil phase obtained above. The addition was made while mixing the solution in a Gifford-Wood Homo-Mixer. The resulting mixture was then passed through a Gaulin Homogenizer (Model 15M-8TA made by A.P.V. Gaulin of Everett, Massachusetts) at a pressure of 1000 psi (6895 kPa). The more times that the emulsion was passed through the high shear homogenizer, the smaller became the size of the droplets. In the present case, the mixture was passed through the homogenizing valve three times.

### (v) Heating the emulsion

Heating was initially conducted under a slight vacuum, for example, 16-20 inches (406-508 mm) of mercury. The temperature of the emulsion rose to about 70°C before the water in the emulsion droplets of aqueous barium titanium citrate began to evaporate through the oil and thence out of the container. Without the vacuum, the water tended to condense on the walls of the container above the emulsion and then drip back into the emulsion, which triggered sedimentation of the aqueous droplets. As the heating continued, a foam developed on the surface of the emulsion. By adjusting the vacuum, the thickness of the foam was maintained at about 1 inch (2.5 cm). The water removal process is endothermic and stabilises the temperature of the emulsion at from 70 to 85°C even though heating is continued. When a substantial proportion of the water in the droplets had been removed, the emulsion temperature began to rise again. When the temperature reached 90°C, the vacuum was discontinued and the heating continued in air at atmospheric pressure. Heating was continued until the temperature of the emulsion reached 130°C, at which point substantially all of the water had evaporated but only a small amount of the mineral oil, if any, had been removed.

The rate of heating was then increased to rapidly raise the temperature to 200°C; the citrate in the emulsion began to break down and decompose at 180°C. The heating rate was then made slow enough (1 to 2°C per minute) that the suspended particles in the emulsion remained in equilibrium with the oil phase and did not flocculate and/or sediment. When the temperature had reached about 270°C essentially all of the low boiling oil (ISOPAR-M) and all of the water had been removed. Heating must not, of course, exceed the boiling and/or decomposition temperature of the surfactant or else the surfactant function of keeping the particles apart will be lost. The OLOA 1200 surfactant used in the present Example begins to decompose at about 270°C.

The "emulsion" was now cooled to room temperature. The material was orange-brown to black in colour depending on the amount of nitric acid which had been used to adjust the pH of the citrate.

The material was an oily slurry and comprised separate particles, each of which derived from a single aqueous emulsion droplet. These dehydrated particles consisted essentially of barium titanium citrate. The particles were suspended in and separated by high-boiling mineral oil and surfactant, the low-boiling paraffinic solvent having been driven off during the heating step; the paraffinic solvent ISOPAR M begins to boil at 207°C and is essentially completely removed by this stage.

### (vi) Charring

A beaker of the oily slurry, or suspension, was placed in a 28,5ℓ (one cubic foot) retort mounted in an oven. The retort was sealed and nitrogen gas was passed through the retort at a rate of 2 standard cubic feet (57 litres) per hour to prevent oxidation. The suspension was heated to 300°C in 15 minutes and then further heated from 300°C to 500°C over 3 hours and held at 500°C for 30 minutes. It was then cooled to room temperature over about 4 hours, still in the nitrogen atmosphere.

As a result of heating to 500°C in this inert atmosphere, both of the organic materials, i.e. the mineral oil and the surfactant, were charred. During this step, the carbon formed advantageously serves to maintain separation between the amorphous particles.

### (vii) Oxidation and Calcination

A layer of char matrix of about 6mm in thickness was spread out on a silica boat and heated in air at a temperature of 700°C for one hour. This produced a thinner layer, the carbon having been removed as CO and CO₂ by the time the char reached 500°C. At 700°C, the constituents of the particles reacted to form barium titanate. The layer of material was further heated to 920°C for thirteen hours to complete the reaction (calcination). The resulting material was found to be crystalline barium titanate, as shown by X-ray diffraction analysis.

The resulting white powder particles were spherical with a narrow particle size distribution.

During the above process for making barium titanate powder, samples of the "dried" emulsion particles in the char and the particles in the calcined powder were subjected to particle size measurements and in some cases were also subjected to analysis by scanning electron microscopy.

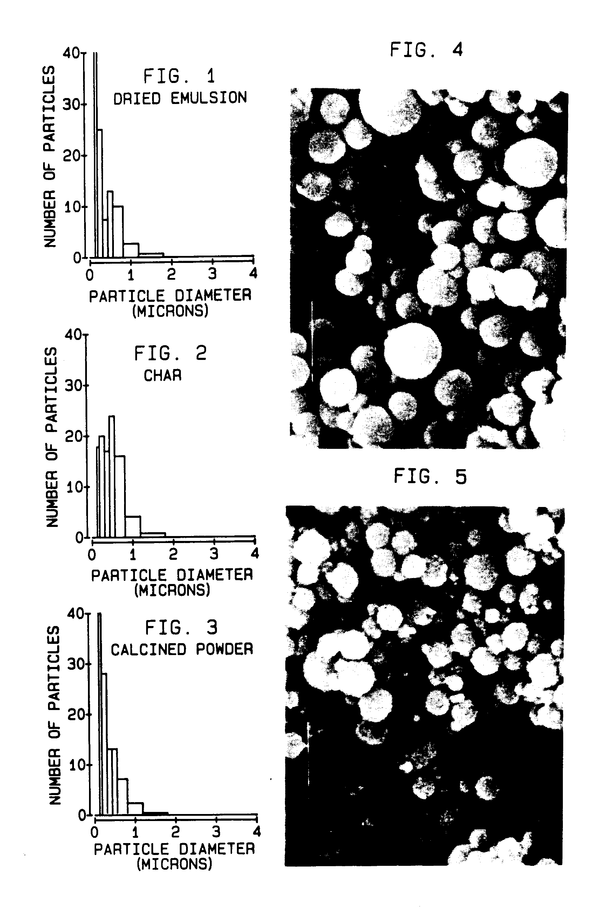

Particle size measurements were made using a MICROTRAC (Trade Mark) Small Particle Analyser (Model No. 7991-3). This instrument uses light scattering to measure various parameters in particulate distributions. It employs laser illumination of a flowing stream of particles, which produces diffraction patterns which are then processed optically and electronically. The instrument cannot detect particles smaller than 0.12 micrometre in diameter. The particle size distributions in samples taken from the heated and "dried" emulsion, from the char, and from the final oxidised/calcined powder product are shown in the form of histograms in Figures 1, 2, and 3 respectively. Electron microscope photographs of particles from the char and from the final powder are shown in Figures 4 and 5 respectively. The photographs were taken at a magnification of x 20,000; the long bar in the photographs represents 1 micrometre.

There is apparently a slight increase in average particle diameter in going from the dried emulsion to the char, which may be due to the carbon coating on the particles of the char. This theory is supported by the fact that the size distribution in the calcined powder is very close to that of the dried emulsion. The calcined powder is somewhat finer than the dried emulsion and char as a result of the expected shrinkage of particles which takes place during calcining. While there is a decrease in individual particle sizes on calcining, the distribution appears to remain the same. In fact, although the mean volume diameter changes slightly in going from dried emulsion to calcined powder, the distribution created by emulsification is retained in the final product.

In the process described above, barium was introduced as barium carbonate, but other water soluble compounds of barium can also be used, such as the citrate, nitrate, or acetate salts. Group I and Group II metals as well as many transition metals commonly found in glass and ceramic compositions form suitable water-soluble salts for use in the present invention. Emulsions according to the present invention have been prepared using barium carbonate, barium acetate, barium nitrate, zirconium acetate, and niobium oxylate as aqueous phase components. Titanium salts other than titanium citrate, such as the tartrate, glycolate, acetate, and lactate, may also be used.

However, solubility in water is not enough for the purposes of the invention. Each water-soluble precursor of the desired powder product must have a higher boiling point and decomposition temperature than the boiling point of water. Only then will it be possible to remove the water from the emulsion droplets by heating without removing the precursor at the same time.

The particular composition of the oil phase used in the process described above is but one of many which can be used in the process of the invention. The oil or water-immiscible organic liquid which is mixed with the aqueous salt solution and homogenized to form the emulsion is preferably itself a mixture of a high boiling oil (e.g. a mineral oil) and a low boiling oil (e.g. an isoparaffin solvent). As the temperature is gradually raised, the free water is first removed and then the lower boiling oil is driven off. The removal of the water is preferably carried out initially at a temperature above the boiling point of water and at or below the boiling point of the low-boiling oil or organic liquid and then at a temperature at or above the boiling point of the low-boiling oil or organic liquid and below the boiling point of the high-boiling oil or organic liquid. The amount of high boiling oil which remains determines the amount of carbon residue which is produced during the subsequent charring step.

It has been shown that the essential feature of the process, that is its ability to control the particle size of the final powder, is retained even when the organic liquid comprises only a high boiling oil, such as a mineral oil. However, the use of only a high boiling oil in the process of the invention requires more extensive heating and renders formulation of the emulsion more critical.

The process of the invention permits a wide latitude in formulating the mixture which, when homogenized, becomes the emulsion. The formulation can be optimised to produce a desired droplet size and thus a desired particle size in the final powder. After the emulsion has been formed an additional quantity of surfactant may be added if required. This additional surfactant does not affect droplet size, but helps to stabilize the suspension during the water removal stage. If too little surfactant is present in the emulsion, the droplets break up and settle to form a sediment.

A substantial quantity of surfactant is added to the oil prior to mixing the oil with the aqueous solution of precursor salts to form the emulsion. Droplet size in the emulsion diminishes according to the quantity of surfactant present.

Surfactant concentrations of less than 1 g/litre render formation of the emulsion difficult and it is generally preferred to use from 2 to 200g of hydrocarbon surfactant per litre of organic liquid. In some cases, concentrations of more than 40g/litre can present difficulties, especially when ultra fine powder particles are required.

The temperature of the emulsion is gradually raised, preferably by supplying heat at a constant rate to the emulsion and preferably under reduced pressure, for example, about half of one standard atmosphere (50.7 kPa). At a temperature of about 70°C, the water from the droplets begins to evaporate; the temperature of the emulsion is maintained at about 70°C until most of the water in the emulsion has been driven off. At this point the emulsion temperature rises and the original emulsion of water and oil has been converted to a suspension of solid particles in oil without any coalescence of the particles taking place.

As indicated above, a preferred oil phase composition for use in the present invention comprises a mixture of a low boiling and a high boiling hydrocarbon. These two hydrocarbons must be mutually soluble and water immiscible. At least the high boiling hydrocarbon must leave a residue of carbon when decomposed in a heated non-oxidizing (reducing) atmosphere and must not break down in a manner which does not produce a carbon residue. The hydrocarbons used preferably contain no metallic elements which might tend to react with and alter the material of the dried droplets, i.e. it is preferred that the residue obtained from charring the hydrocarbon(s) is entirely carbon.

The process parameters for the charring step are not very critical. It is important, however, not to exceed the temperature at which the precursor compounds in the dried droplets will begin to inter-react because, if this occurs, carbon may be incorporated into the reaction product. Such incorporation of carbon modifies the composition and degrades the properties of the final powder product and detracts from an otherwise highly repeatable and controllable process.

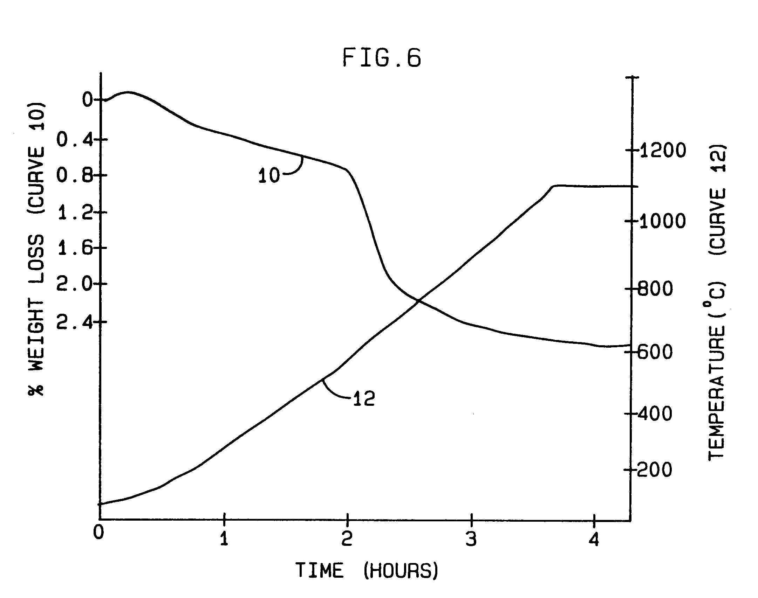

In the process described above for making barium titanate powder, a sample of the char was heated and oxidized at a temperature of only 500°C and allowed to cool. The oxidized material, a white amorphous powder from which the carbon had been removed, was then heated at a rate of 5°C/minute in air while measuring the weight of the powder. The change in powder weight as a function of time is plotted as curve 10 in Figure 6, while the temperature as a function of time is plotted as curve 12. It can be seen from these curves that the major calcining reaction begins at about 550°C. During calcining over the temperature range 550°C to 700°C, the weight loss of the powder amounted to 1.1% and the amorphous material of the particles was converted to crystalline barium titanate, as shown by X-ray diffraction analysis. Thus oxidation and removal of the carbon is essentially complete before the onset of calcination and crystallization.

The parameters must be controlled during the oxidation step. The main precaution necessary is to adjust the density or bulk of the char to be oxidized and/or adjust the rate of heating or the amount of oxygen available in order to maintain control of the temperature at which the exothermic reaction takes place. If the reaction is uncontrolled, the rise in temperature accelerates the reaction and an autocatalytic reaction follows. If the exothermic reaction gets out of control, calcination can begin prior to complete oxidation, leading to carbon contamination of the product.

### Example 2

### Preparation of zirconium acetate

200 g of glacial acetic acid was placed in a round-bottomed flask and 100 g of zirconium n-propoxide was added. The mixture was stirred and refluxed for about 2 hours. A precipitate formed which tended to stop the stirring action, but stirring was maintained to prevent overheating.

The refluxed mixture was cooled and filtered through a medium porosity filter to remove the solids. The solids were vacuum dried at 60°C for several hours to give about 65 g of dry zirconium acetate.

This material can be assayed for ZrO₂ content by heating in air at a temperature of 800°C for 30 minutes. The ZrO₂ content will be about 45% by weight.

The zirconium acetate may be incorporated into the aqueous phase of an emulsion according to the invention to serve as a precursor for a powder product comprising zirconium. For example, if it is the only precursor in the aqueous phase, the subsequent heating steps by which the emulsion is dried, charred, and oxidized will produce a fine powder of ZrO₂. Alternatively, zirconium acetate can be used instead of titanium citrate in the process of Example 1 for making barium titanate powder; the powder product produced will then be barium zirconate.

### Example 3

### Preparation of a PLZT citrate solution

29.69 g of La(NO₃) . 6H₂O was dissolved in 160 ml of double deionized water in a beaker. In another beaker 110 g of anhydrous citric acid was dissolved in 100 ml of water and the pH of the solution was adjusted to 7.1 by the addition of NH₄OH. The lanthanum and citrate solutions were then mixed in a 1500 ml beaker and 13.70/X g of the titanium citrate solution obtained from Example 1 was added; X is the weight fraction of TiO₂ in the titanium citrate solution, as determined by assay. The pH of the mixture was adjusted to 7.4 by adding NH₄OH. 112.23 g of PbO (litharge) was also added which made the mixture cloudy. The mixture was stirred and heated at a temperature of 80°C for 30 minutes to render it clear. A further addition of 49.29/Y g of the zirconium acetate obtained from Example 2 was effected slowly, while maintaining the pH of the mixture at about 7 by adding NH₄OH as required; Y is the weight fraction of ZrO₂ in the zirconium acetate. Water may be added to adjust the volume to 1000 ml; the final pH will be in the range 7.0 to 7.4

### Example 4

### Preparation of magnesium borate powder

1.2 moles of citric acid was dissolved in doubly deionized water. 1.2 mole of MgCO₃ was then added to the solution and reacted therewith to form a 1.0 M solution of MgHC₆H₅O₇. While continuing to heat and stir the solution, 0.8 mole of H₃BO₃ was added at a temperature of 40°C until a clear green liquid was obtained.

The solution was then emulsified in the immiscible hydrocarbon oil phase described in Example 1, except that 40 g (rather than 20 g) of OLOA 1200 surfactant were used per litre of oil. The mixture was emulsified by the method described in Example 1 and then heated to remove the water following the procedure described in Example 1 for making barium titanate powder. Charring was carried out at 500°C. The char was oxidized at a temperature of 715°C for 1 hour and then calcined at 920°C for 16 hours in a standard air atmosphere. A fine powder comprising Mg₃B₂O₆ was produced in which 90% of the particles had a diameter of less than 0.61 micrometre and the average particle size was 0.32. A few large particles in the 1 to 20 micrometre range appeared to be agglomerates which had formed during calcining. A slightly lower calcining temperature or a shorter calcining time or both are expected to eliminate such agglomerations.

## Claims

1. A process for making a material in powder form, which comprises
(a) mixing an aqueous solution of a compound or compounds capable of conversion to the desired final product, the compound/compounds having a higher boiling point and decomposition temperature than the boiling point of water under the conditions used in step (b) below, and a water-immiscible organic liquid to form a water/organic liquid emulsion in which the aqueous solution is the disperse phase.
(b) heating the emulsion from step (a) to evaporate and remove the water and a portion of the organic liquid to give a suspension of solid particles constituting the precursor compound(s) in the remaining organic liquid,
(c) heating the suspension from step (b) in a non-oxidising atmosphere to char the organic liquid and form a carbon char matrix having the solid particles dispersed therein, and
(d) heating the product of step (c) in an oxidising atmosphere to oxidise and remove the carbon of the matrix and to leave the solid particles in the form of a powder.

2. A process according to claim 1, in which step (b) is initially carried out at a temperature above the boiling point of water and at or below the boiling point of the organic liquid and then at a temperature at or above the boiling point of the organic liquid.

3. A process according to claim 1, in which the organic liquid comprises a low-boiling organic liquid and a high-boiling organic liquid which are mutually soluble.

4. A process according to claim 3, in which step (b) is initially carried out at a temperature above the boiling point of water and at or below the boiling point of the low-boiling organic liquid and then at a temperature at or above the boiling point of the low-boiling organic liquid and below the boiling point of the high-boiling organic liquid.

5. A process according to any of claims 1 to 4, in which the organic liquid is metal-free.

6. A process according to any of claims 1 to 5, in which the organic liquid further comprises a hydrocarbon surfactant.

7. A process according to claim 6, in which the hydrocarbon surfactant is present in an amount of from 2 to 200 g/litre of organic liquid.

8. A process according to any of claims 1 to 7, in which step (b) is carried out under reduced pressure.

9. A process according to any of claims 1 to 8, in which the maximum temperature employed during step (b) is below the boiling points and/or decomposition temperatures of the precursor compound(s) and the surfactant.

10. A process according to any of claims 1 to 9, in which steps (c) and (d) are carried out at temperatures below that at which the precursor compounds inter-react.

11. A process according to any of claims 1 to 10, in which the powder obtained from step (d) is additionally calcined at a temperature above that at which step (d) was carried out.

## Patentansprüche

1. Verfahren zum Herstellen eines Materials in Pulverform, welches umfaßt
a) Vermischen einer wäßrigen Lösung von einer Verbindung oder Verbindungen, die in das gewünschte Endprodukt umgewandelt werden können, wobei die Verbindung/Verbindungen einen höheren Siedepunkt und eine höhere Zersetzungstemperatur als den Siedepunkt von Wasser unter den in Schritt (b) unten verwendeten Bedingungen haben, und einer nicht mit Wasser mischbaren organischen Flüssigkeit, um eine Wasser/organische Flüssigkeit-Emulsion zu bilden, in der die wäßrige Lösung die dispergierte Phase ist.
b) Erwärmen der Emulsion aus Schritt (a) um das Wasser und einen Teil der organischen Flüssigkeit zu verdampfen und zu entfernen, um eine Suspension aus festen Teilchen zu ergeben, welche die Vorläuferverbindung(en) in der zurückbleibenden organischen Flüssigkeit bilden,
c) Erwärmen der Suspension aus Schritt (b) in einer nicht-oxidierenden Atmosphäre, um die organische Flüssigkeit zu verkohlen und eine Kohlenstoffmatrix zu bilden, welche die festen Teilchen darin dispergiert hat, und
d) Erwärmen des Produkts aus Schritt (c) in einer oxidierenden Atmosphäre, um den Kohlenstoff der Matrix zu oxidieren und zu entfernen und um die festen Partikel in der Form eines Pulvers zu hinterlassen.

2. Verfahren nach Anspruch 1, worin Schritt (b) anfänglich bei einer Temperatur oberhalb des Siedepunkts von Wasser und bei oder unter dem Siedepunkt der organischen Flüssigkeit und danach bei einer Temperatur bei oder oberhalb des Siedepunkts der organischen Flüssigkeit ausgeführt wird.

3. Verfahren nach Anspruch 1, worin die organische Flüssigkeit eine niedrigsiedende organische Flüssigkeit und eine hochsiedende organische Flüssigkeit umfaßt, welche ineinander gegenseitig löslich sind.

4. Verfahren nach Anspruch 3, worin der Schritt (b) anfänglich bei einer Temperatur oberhalb des Siedepunkts von Wasser und bei oder unter dem Siedepunkt der niedrigsiedenden organischen Flüssigkeit und danach bei einer Temperatur bei oder oberhalb des Siedepunkts der niedrigsiedenden organischen Flüssigkeit und unterhalb des Siedepunkts der hochsiedenden organischen Flüssigkeit ausgeführt wird.

5. Verfahren nach einem der Ansprüche 1 bis 4, worin die organische Flüssigkeit metallfrei ist.

6. Verfahren nach einem der Ansprüche 1 bis 5, worin die organische Flüssigkeit weiter ein Kohlenwasserstoff-Tensid umfaßt.

7. Verfahren nach Anspruch 6, worin das Kohlenwasserstoff-Tensid in einer Menge von 2 bis 200 g/l der organischen Flüssigkeit vorhanden ist.

8. Verfahren nach einem der Ansprüche 1 bis 7, worin Schritt (b) unter vermindertem Druck ausgeführt wird.

9. Verfahren nach einem der Ansprüche 1 bis 8, worin die maximale Temperatur, die im Schritt (b) eingesetzt wird, unterhalb der Siedepunkte und/oder Zersetzungstemperaturen der Vorläuferverbindung(en) und des Tensids liegt.

10. Verfahren nach einem der Ansprüche 1 bis 9, worin die Schritte (c) und (d) bei Temperaturen unterhalb derjenigen, bei der die Vorläuferverbindungen miteinander reagieren, durchgeführt werden.

11. Verfahren nach einem der Ansprüche 1 bis 10, worin das im Schritt (d) erhaltene Pulver zusätzlich bei einer Temperatur oberhalb der, bei der der Schritt (d) ausgeführt wurde, calciniert wird.

## Revendications

1. Procédé de préparation d'une substance sous forme de poudre, dans lequel
(a) on mélange une solution aqueuse d'un composé ou de composés capable(s) de se transformer en le produit final désiré, le ou les composé(s) ayant un point d'ébullition et une température de décomposition supérieurs au point d'ébullition de l'eau dans les conditions indiquées dans l'étape (b) ci-dessous, et un liquide organique non miscible à l'eau pour former une émulsion eau/liquide organique dans laquelle la solution aqueuse est la phase dispersée,
(b) on chauffe l'émulsion issue de l'étape (a) pour évaporer et éliminer l'eau et une partie du liquide organique pour donner une suspension de particules solides constituant le ou les composé(s) précurseur(s) dans le liquide organique restant,
(c) on chauffe la suspension issue de l'étape (b) dans une atmosphère non oxydante pour carboniser le liquide organique et pour former une matrice de charbon de carbone dans laquelles sont dispersées les particules solides, et
(d) on chauffe le produit de l'étape (c) dans une atmosphère oxydante pour oxyder et séparer le carbone de la matrice et pour laisser les particules solides sous forme d'une poudre.

2. Procédé selon la revendication 1, dans lequel on réalise l'étape (b) d'abord à une température supérieure au point d'ébullition de l'eau et égale ou inférieure au point d'ébullition du liquide organique puis à une température égale ou supérieure au point d'ébullition du liquide organique.

3. Procédé selon la revendication 1, dans lequel le liquide organique comprend un liquide organique de bas point d'ébullition et un liquide organique de point d'ébullition élevé qui sont mutuellement solubles.

4. Procédé selon la revendication 3, dans lequel on réalise l'étape (b) d'abord à une température supérieure au point d'ébullition de l'eau et égale ou inférieure au point d'ébullition du liquide de bas point d'ébullition, puis à une température égale ou supérieure au point d'ébullition du liquide de bas point d'ébullition et inférieure au point d'ébullition du liquide de point d'ébullition élevé.

5. Procédé selon l'une quelconque des revendications 1 à 4, dans lequel le liquide organique ne contient pas de métal.

6. Procédé selon l'une quelconque des revendications 1 à 5, dans lequel le liquide organique contient en outre un agent tensio-actif hydrocarboné.

7. Procédé selon la revendication 6, dans lequel l'agent tensio-actif est présent en une quantité de 2 à 200 g/litre de liquide organique.

8. Procédé selon l'une quelconque des revendications 1 à 7, dans lequel l'étape (b) est réalisée sous pression réduite.

9. Procédé selon l'une quelconque des revendications 1 à 8, dans lequel la température maximale utilisée au cours de l'étape (b) est inférieure aux points d'ébullition et/ou aux températures de décomposition du ou des composé(s) précurseur(s) et de l'agent tensio-actif.

10. Procédé selon l'une quelconque des revendications 1 à 9, dans lequel les étapes (c) et (d) sont réalisées à des températures inférieures à celle à laquelle les composés précurseurs réagissent entre eux.

11. Procédé selon l'une quelconque des revendications 1 à 10, dans lequel la poudre obtenue à l'issue de l'étape (d) subit une calcination supplémentaire à une température supérieure à celle à laquelle a été réalisée l'étape (d).
